Europäisches Patentamt

(19) **European Patent Office**

**Office européen des brevets**

(11) Numéro de publication : **0 032 344 B1**

(12) **FASCICULE DE BREVET EUROPÉEN**

(45) Date de publication du fascicule du brevet :
14.09.83

(51) Int. Cl.³ : **A 61 K 35/54, A 61 K 45/06**

(21) Numéro de dépôt : **80401866.1**

(22) Date de dépôt : **24.12.80**

(54) Lyophilisat d'oeuf de caille pour le traitement des maladies immuno-allergiques.

(30) Priorité : **28.12.79 FR 7931997**

(43) Date de publication de la demande :
**22.07.81 Bulletin 81/29**

(45) Mention de la délivrance du brevet :
**14.09.83 Bulletin 83/37**

(84) Etats contractants désignés :
**AT BE CH DE GB IT LI LU NL SE**

(56) Documents cités :
FR A 2 356 426

LA RECHERCHE, vol. 10, n° 104, octobre 1979,
J. BENVENISTE : « Guérir l'allergie », pages
1012-1013

REMINGTON'S PHARMACEUTICAL SCIENCES
(1975) MACK PUBL. CO. USA. page 1483

ARZNEIFORMENLEHRE (1976) P.H. LIST. Wissenschaftliche Verlagsgesellschaft mbH. STUTT-
GART. pages 247, 248, 252

Le dossier contient des informations techniques présentées postérieurement au dépôt de la demande et
ne figurant pas dans le présent fascicule.

(73) Titulaire : **Truffier, Jean-Claude**
**67, rue Thriers**
**F-17300 Rochefort (FR)**

**Pinon, Claude**
**108, Boulevard de Courcelles**
**F-75017 Paris (FR)**

(72) Inventeur : **Truffier, Jean-Claude**
**67, rue Thriers**
**F-17300 Rochefort (FR)**
Inventeur : **Pinon, Claude**
**108, Boulevard de Courcelles**
**F-75017 Paris (FR)**

(74) Mandataire : **Hufénus, Christiane et al**
**CABINET BEAU DE LOMENIE 55, rue d'Amsterdam**
**F-75008 Paris (FR)**

# 0 032 344

## Lyophilisat d'œuf de caille pour le traitement des maladies immuno-allergiques

L'expérimentation clinique et biologique de l'œuf de caille souche B Mina, rapportée dans « La Clinique » du 15 mars 1978 et dans l'article de « La Recherche » vol. 10, n° 104, octobre 1979, pages 1 012-3, a établi l'efficacité du traitement des maladies immuno-allergiques par administration orale d'œuf de caille cru. D'autre part, le brevet français 2.356.426 décrit un procédé de préparation d'une fraction ovomucoïde à activité protéasique à partir du blanc de l'œuf de caille, fraction qui a les mêmes propriétés anti-allergiques que celles de l'œuf cru. On a trouvé selon l'invention que, pour le traitement des maladies immuno-allergiques on peut remplacer l'œuf cru par un lyophilisat d'œuf de caille souche B Mina, ou d'autres souches, le lyophilisat ayant le même effet thérapeutique tout en présentant par rapport à l'œuf cru des avantages sur le plan de l'absorption et de la conservation. De plus, on a montré que les malades recevant le lyophilisat selon l'invention ne présentaient aucun des signes d'intolérance relevés chez les malades traités par l'œuf de caille cru.

Le procédé de préparation du lyophilisat est, par exemple, le suivant : le contenu de chaque œuf est prélevé séparément par aspiration avec une seringue stérile reliée à une pompe à vide. Il est pasteurisé à 64 °C pendant 5 min. Il subit ensuite une lyophilisation par abaissement de la température à − 40 °C sous pression inférieure à la pression atmosphérique, puis réchauffement à + 40 °C pour éliminer l'eau. Le lyophilisat d'œuf de caille a subi les contrôles bactériologiques et mycologiques réglementaires pour les ovo-produits, avant d'être utilisé. Il a été conditionné en sachets contenant 6 g de poudre correspondant à 3 œufs lyophilisés.

Tout autre procédé classique pour la lyophilisation des œufs peut être utilisé.

### Etude toxicologique

Le lyophilisat a fait l'objet d'une étude de toxicologie aiguë et chronique chez le rat, qui a démontré l'absence de toxicité sur cette espèce.

De plus, on a étudié l'effet sur le rat et la souris sensibilisés à l'ovalbumine de poule. Il a été noté l'absence d'action antihistaminique, mais une diminution de la production des IgE spécifiques.

### Etude clinique et biologique

L'effet thérapeutique du lyophilisat a été étudié chez l'homme et chez le chien allergiques soit comme premier traitement, soit comme traitement de rappel après des cures d'œufs crus chez des sujets rechutant après une amélioration de durée variable.

### 1. Expérimentation chez l'homme

#### Indications thérapeutiques

Les principales indications sont :

— allergies de type I : eczéma atopique, asthme allergique, rhinite apériodique et saisonnière, conjonctivite, trachéite, urticaire, œdème de Quincke, allergies digestives, quels que soient les allergènes responsables,
— allergies de types III et IV : allergies solaires,
— autres affections : des améliorations notables des différentes affections suivantes ont été notées au cours de traitements d'allergies par le lyophilisat d'œuf de caille : maladies auto-immunes (P.C.E., lupus érythémateux), ulcus, herpès récidivant, hyper-uricémies, déficit sérique ou sécrétoire en antiprotéases, cicatrisation, pelade, calvitie, vieillissement cellulaire, cirrhose et séquelles d'hépatite, baisse de la libido et impuissance, diabète, céphalées, altération de l'état général.

#### Posologie

Chaque traitement comporte deux cures de 9 jours séparées d'un arrêt de 9 jours avec :

— prise de 8 g de lyophilisat par jour les 3 premiers jours,
— prise de 10 g de lyophilisat par jour les 3 jours suivants,
— prise de 12 g de lyophilisat par jour les 3 derniers jours.

Le lyophilisat peut être absorbé avec du lait froid ou un peu de yaourt, purée, jus de fruit et sans alcool ni boisson chaude.
Dans certains cas, il est nécessaire d'augmenter la posologie en conseillant :

— soit 2 à 4 g de lyophilisat en plus par jour lors de la deuxième cure,
— soit de faire une mini-cure avec :

8 g de lyophilisat par jour pendant 2 jours,

10 g de lyophilisat par jour les 2 jours suivants,

12 g de lyophilisat par jour les 2 derniers jours.

En cas de rechute, il est conseillé une cure de rappel de 9 jours. Les patients doivent rester en contact avec leurs allergènes pendant la durée du traitement.

Surveillance biologique

Afin de surveiller la tolérance et l'efficacité du lyophilisat, les malades ont subi, avant et après le traitement, les examens suivants :

— numération, formule sanguine,
— IgE sériques, totales et spécifiques,
— test de dégranulation des basophiles à l'œuf de caille et aux allergènes responsables,
— tests cutanés et bilan fonctionnel.

Efficacité

L'effet thérapeutique apparaît généralement chez l'homme entre le 30e et le 90e jour après le début du traitement. Dans les allergies de type I, près de 80 % des patients présentent une amélioration des symptômes qui sont atténués de 30 à 100 %. L'amélioration ne porte que sur le facteur allergique. Les composantes infectieuses, psychiques, hormonales et irritatives ne sont pas influencées par le traitement.

Effets secondaires

L'effet secondaire le plus fréquemment observé est une légère accentuation de la maladie survenant le plus souvent entre le 3e et le 6e jour de traitement.

D'autres effets secondaires sont plus rares, diarrhée, céphalées, expectoration, et cessent à l'arrêt du traitement.

Effets biologiques après 3 mois :

— tests cutanés : ils sont inchangés ou parfois légèrement accentués,
— éosinophiles sanguins : ils sont inchangés ou augmentés, puis diminués,
— IgE sériques spécifiques : elles sont inchangées, parfois augmentées, puis abaissées,
— IgE totales : elles subissent des variations notables (élévation ou abaissement) non corrélées avec l'amélioration clinique,
— test de dégranulation des basophiles humains (technique BENVENISTE) : on observe une diminution significative du taux de positivité dans 75 % des cas, après parfois une accentuation passagère. Des tests négativés après traitement efficace par le lyophilisat se sont repositivés en cas de rechute.

2. Expérimentation sur le chien

Posologie

Chaque traitement a comporté deux cures de 9 jours séparées d'un arrêt de 9 jours, avec, lors de chaque cure, ingestion de 8 g de lyophilisat par jour.

Indications thérapeutiques

Les chiens traités étaient atteints soit d'eczéma, avec ou sans pelade, soit d'allergies respiratoires avec rhinite, trachéite et asthme.

Efficacité

Dans l'eczéma, l'amélioration est en général rapide, dès le 4e jour, avec arrêt du prurit, cicatrisation, puis repousse du poil.

Dans l'allergie respiratoire, une aggravation est possible vers le 3e jour, suivie d'une amélioration qui atteint son maximum dès la fin de la deuxième cure.

En résumé, le lyophilisat a un effet thérapeutique comparable à l'œuf de caille cru avec moins d'effets secondaires, ce qui permet d'augmenter la posologie. On retrouve, comme pour l'œuf cru :

**0 032 344**

1. la nécessité du contact avec les allergènes responsables pendant le traitement,
2. la possibilité d'aggravation passagère suivie d'une amélioration plus ou moins retardée,
3. la possibilité de rechute avec la posologie utilisée.

L'œuf lyophilisé semble agir en modifiant la réponse immunitaire, en provoquant une immuno-stimulation suivie après un mois d'une diminution de la réponse immune spécifique.
Il est donc possible d'envisager son action par :

1. stimulation des lymphocytes T suppresseurs,
2. inhibition de la production des IgE par l'action des antiprotéases présentes dans le lyophilisat,
3. effet de tachyphylaxie.

En conclusion, le lyophilisat d'œuf de caille a une action thérapeutique sur les allergies de types I, III, IV, et sur des affections non allergiques énumérées ci-dessus, chez l'homme et chez l'animal, à la posologie de 2 à 12 g par jour, en cures de 9 jours suivies d'arrêt de 9 jours.
Il est possible d'ajouter au lyophilisat :

— des substances lipotropes (méthionine, etc.) pour améliorer la tolérance hépatique,
— des vitamines,
— d'autres substances anti-allergiques pour atténuer les effets secondaires et apporter un soulagement immédiat.

L'invention concerne également des compositions dans lesquelles le lyophilisat est associé à un allergène, à doses adaptées à la tolérance des malades.

## Revendications

1. Compositions pharmaceutiques destinées à l'administration par voie orale, pour utilisation dans le traitement des maladies immuno-allergiques, caractérisées en ce qu'elles contiennent le lyophilisat d'œufs de caille à la dose unitaire de 2 à 10 g, qui correspond à une dose de 1 à 5 œufs lyophilisés.
2. Compositions pharmaceutiques selon la revendication 1, caractérisées en ce qu'elles contiennent le lyophilisat d'œuf de caille associé à l'allergène responsable de la maladie à traiter.

## Claims

1. Pharmaceutical compositions designed to be administered orally, for use in the treatment of immuno-allergic diseases, characterised in that they contain the lyophilisate from quails eggs in the unit dose of 2 to 10 g, corresponding to a dose of 1 to 5 lyophilized eggs.
2. Pharmaceutical compositions according to claim 1, characterised in that they contain the lyophilisate from quails eggs in combination with the allergen responsible for the disease to be treated.

## Ansprüche

1. Pharmazeutische Zusammensetzungen für orale Verabreichung zur Verwendung bei der Behandlung von immunoallergischen Krankheiten, dadurch gekennzeichnet, daß sie das Lyophilisat von Wachteleiern in einer Einheitsdosis von 2 bis 10 g enthalten, was einer Dosis von 1 bis 5 lyophilisierten Eiern entspricht.
2. Pharmazeutische Zusammensetzungen nach Anspruch 1, dadurch gekennzeichnet, daß sie das Lyophilisat von Wachteleiern zusammen mit dem für die zu behandelnde Krankheit verantwortlichen Allergen enthalten.

4